# EUROPÄISCHE PATENTSCHRIFT

(11) **EP 1 743 882 B1**
(45) Veröffentlichungstag und Bekanntmachung des Hinweises auf die Patenterteilung: **02.02.2011**
(21) Anmeldenummer: 06013571.2
(22) Anmeldetag: 30.06.2006
(51) Int. Cl.: C07C 263/10

(54) **Verfahren zur Herstellung von Isocyanaten**
Process for the preparation of isocyanates
Procédé de préparation d'isocyanates

(30) Priorität: 13.07.2005 DE 10532663
(43) Veröffentlichungstag der Anmeldung: 17.01.2007
(73) Patentinhaber: Bayer MaterialScience AG, 51368 Leverkusen (DE)
(72) Erfinder: Bulan, Andreas, 40764 Langenfeld (DE); Weber, Rainer, Dr., 51519 Oldenthal (DE); Lorenz, Wolgang, Dr., 41540 Dormagen (DE); Moormann, Gerhard, 25541 Brunsbüttel (DE); Kämper, Friedhelm, Dr., 47807 Krefeld (DE); Keggenhoff, Berthold, Dr., 47839 Krefeld (DE)

(56) Entgegenhaltungen:
- WO-A1-97/24320
- WO-A1-2004/014845
- WO-A1-2006/095761
- WO-A2-02/18675
- WO-A2-2004/037718

## Beschreibung

Die vorliegende Erfindung betrifft ein integriertes Verfahren zur Herstellung von Isocyanaten aus Phosgen und wenigstens einem Amin und elektrochemische Oxidation des dabei anfallenden Chlorwasserstoffs zu Chlor, wobei das Chlor zur Herstellung des Phosgens rückgeführt wird.

Bei der Herstellung vieler organischer Verbindungen sowie bei der Herstellung von Rohstoffen für die Produktion von Polymeren wird in der Produktionskette sehr häufig Chlor als Oxidationsmittel eingesetzt. Als Nebenprodukt entsteht dabei häufig Chlorwasserstoff. Zum Beispiel wird Chlor in der Isocyanatherstellung eingesetzt, wobei als Nebenprodukt Chlorwasserstoff gebildet wird. Die weitere Nutzung des Chlorwasserstoffs kann z.B. durch Vermarktung der wässrigen Lösung (Salzsäure) oder durch Verwendung bei Synthesen anderer chemischer Produkte erfolgen. Die anfallenden Mengen Chlorwasserstoff können jedoch nicht immer vollständig vermarktet oder für andere Synthesen eingesetzt werden. Außerdem kann Chlorwasserstoff für Synthesen nur dann eingesetzt werden, wenn er zuvor entsprechend gereinigt wird. Andererseits ist die Vermarktung meist nur dann wirtschaftlich, wenn der Chlorwasserstoff bzw. die Salzsäure nicht über weite Wege transportiert werden müssen. Eine der gängigsten Nutzungsmöglichkeiten für den anfallenden Chlorwasserstoff ist die Verwendung als Rohstoff bei der PVC-Herstellung, bei dem die Oxychlorierung von Ethylen mit Chlorwasserstoff zu Ethylendichlorid erfolgt. Die Entsorgung des Chlorwasserstoffes, z.B. durch Neutralisation mit Lauge, ist aus wirtschaftlicher und ökologischer Sicht unattraktiv.

Ein Recyclingverfahren für den Chlorwasserstoff und die Rückführung des Chlors und/oder des Wasserstoffs in den Produktionsprozess, in dem der Chlorwasserstoff anfällt, ist daher die angestrebte Verfahrensweise. Zu den Recyclingverfahren zählen die katalytische Oxidation von Chlorwasserstoff, z.B. nach dem Deacon-Verfahren, die Elektrolyse von gasförmigem Chlorwasserstoff sowie die Elektrolyse einer wässrigen Lösung von Chlorwasserstoff (Salzsäure). So ist z.B. aus WO 04/14845 ein integriertes Verfahren zur Herstellung von Isocyanaten und katalytischer Oxidation von Chlorwasserstoff nach dem Deacon-Verfahren und aus WO 97/24320 ein integriertes Verfahren zur Herstellung von Isocyanaten und Gasphasenelektrolyse von Chlorwasserstoff bekannt.

Eine Übersicht über elektrochemische Recycling-Verfahren wird in dem Artikel "Chlorine Regeneration from Anhydrous Hydrogen Chloride" von Dennie Turin Mah, veröffentlicht in "12th International Forum Electrolysis in Chemical Industry - Clean and Efficient Processing Electrochemical Technology for Synthesis, Separation, Recycle and Environmental Improvement, October 11-15, 1998, Sheraton Sand Key, Clearwater Beach, FL", gegeben.

Die katalytische Chlorwasserstoff-Oxidation nach dem Deacon-Prozess als Recycling-Verfahren, wie z.B. in WO 04/014845 beschrieben, hat einige verfahrenstechnische Nachteile. So kann die heterogen katalysierte Chlorwasserstoff-Oxidation nur beschränkt auf unterschiedliche Lastzustände eingestellt werden. Der Deacon-Prozess ist deutlich empfindlicher gegenüber Laständerungen als die Elektrolyse. Auch eine Änderung der Kapazität einer großtechnischen Anlage zur katalytischen Chlorwasserstoff-Oxidation ist mit Aufwand verbunden.

Ein weiterer Nachteil der katalytischen Chlorwasserstoff-Oxidation ist, dass der für die Reaktion eingesetzte Katalysator außerordentlich empfindlich gegen Verunreinigungen im Chlorwasserstoff ist. Infolge eines Aktivitätsverlusts des Katalysators sinkt die Recycling-Kapazität drastisch. Gleichzeitig wird durch den geringeren Umsatz der Chlorwasserstoff-Oxidation im Reaktor die Aufarbeitung der aus dem Reaktor austretenden Reaktionsgase (Sauerstoff, Chlorwasserstoff, Chlor, Wasser) aufwendiger. Dies reduziert insgesamt die Wirtschaftlichkeit des katalytischen Oxidationsverfahrens deutlich.

In WO 97/24320 bzw. EP 876 335 A wird ein Verfahren beschrieben, bei dem der bei der Isocyanatherstellung anfallende Chlorwasserstoff mittels Gasphasenelektrolyse zu Chlor umgewandelt wird und das Chlor in die Phosgenherstellung zur Herstellung des Isocyanats rückgeführt wird. Im speziellen Fall der Herstellung von Toluylendiisocyanat (TDI) wird außerdem Wasserstoff in die Herstellung von Toluylendiamin (TDA) rückgeführt. Die Umwandlung von Chlorwasserstoff in Chlor durch Elektrolyse in der Gasphase ist großtechnisch noch nicht erprobt und hat den Nachteil, dass die großtechnische Durchführung erhöhte technische Anforderungen an die Bauteile der Anlage, beispielsweise hinsichtlich ihrer Druckbeständigkeit, stellt und mit erhöhtem sicherheitstechnischem Aufwand verbunden ist. Ein weiterer Nachteil ist, dass bei unvollständigem Umsatz des Chlorwasserstoffs ein weiterer Verfahrensschritt durchgeführt werden muss, bei dem das gebildete Chlor von überschüssigem Chlorwasserstoff getrennt wird. Gemäß EP 1 106 714 A wird daher zur Verbesserung des Umsatzes der Gasphasenelektrolyse dem gasförmigen Chlorwasserstoff Sauerstoff zugegeben. Nachteilig hierbei ist, dass bei unvollständigem Umsatz des Sauerstoffs das gebildete Chlor von Chlorwasserstoff und zusätzlich von Sauerstoff befreit werden muss, zum Beispiel durch Totalverflüssigung.

Darüber hinaus können gemäß WO 97/24320 u.a. sogenannte Festelektrolytsysteme, z.B. Nafion®-Membrane, eingesetzt werden, wobei die Anode und Kathode jeweils auf einer Seite der Ionenaustauschermembran anliegen. Die Anode und Kathode können z.B. Gasdiffusionselektroden sein. Alternativ kann das katalytisch aktive Material, das als Anode bzw. Kathode fungiert, in die Ionenaustauschermembran eingearbeitet oder auf die Ionenaustauschermembran aufgebracht sein. Nachteilig daran ist, dass bei einer Kontamination oder Beschädigung der Ionenaustauschermembran oder des katalytisch aktiven Materials die gesamte Einheit, bestehend aus Ionenaustauschermembran und katalytisch aktivem Material der Elektroden, ausgetauscht werden muss.

Die elektrochemische Oxidation einer wässrigen Lösung von Chlorwasserstoff unter Verwendung einer Gasdiffusionselektrode als Kathode ist z.B. in WO 00/73538 und WO 02/18675 beschrieben. Dabei wird als Katalysator für die Sauerstoffreduktion an der Kathode Rhodiumsulfid eingesetzt. Gemäß WO 02/18675 ist dieser Katalysator weitgehend beständig gegen organische Bestandteile, die als Verunreinigungen in der Salzsäure vorliegen können und z.B. aus vorgeschalteten Syntheseschritten stammen. Die organischen Bestandteile gelangen über die Ionenaustauschermembran aus dem Anodenraum in den Kathodenraum. Organische Verunreinigungen führen bei längerer Betriebszeit der Elektrolyse zu einem Spannungsanstieg, was die Wirtschaftlichkeit des Verfahrens negativ beeinflusst. Zur Entfernung organischer Bestandteile wird in WO 02/18675 eine Reinigung der Salzsäure mittels Aktivkohle und ggf. zusätzlich mittels eines Austauscherharzes, z.B. eines Molekularsiebes, vorgeschlagen.

Aufgabe der vorliegenden Erfindung ist es daher, ein Verfahren zur Herstellung von Isocyanaten unter Recycling des bei der Isocyanatherstellung anfallenden Chlorwasserstoffs zur Verfügung zu stellen, welches einfach und sicher betrieben werden kann. Insbesondere soll das Verfahren eine schnelle Inbetriebnahme und Außerbetriebnahme sowie einen einfachen Betrieb bei unterschiedlichen Lastzuständen gewährleisten. Auch eine Kapazitätssteigerung soll einfach möglich sein.

Gegenstand der Erfindung ist ein Verfahren zur Herstellung von Isocyanaten, umfassend die folgenden Schritte:
a) Herstellung von Phosgen durch Umsetzung von Chlor mit Kohlenmonoxid
b) Umsetzung des gemäß Schritt (a) gebildeten Phosgens mit wenigstens einem organischen Amin unter Bildung wenigstens eines Isocyanats und von Chlorwasserstoff
c) Abtrennung und Aufarbeitung der in Schritt (b) gebildeten Isocyanate
d) Abtrennung des gemäß Schritt (b) gebildeten Chlorwasserstoffs
e) Herstellung einer wässrigen Lösung des Chlorwasserstoffs
f) Reinigung der wässrigen Lösung von Chlorwasserstoff durch Strippung mittels Wasserdampf, so dass der Anteil organischer Lösungsmittel maximal 0,05 Gew.-% beträgt,
g) Elektrochemische Oxidation wenigstens eines Teils der wässrigen Chlorwasserstofflösung zu Chlor
h) Rückführung wenigstens eines Teils des gemäß Schritt (g) hergestellten Chlors in die Herstellung von Phosgen gemäß Schritt (a).

Das erfindungsgemäße Verfahren ist ein integriertes Verfahren zur Herstellung von Isocyanaten und der Elektrolyse einer wässrigen Lösung aus Chlorwasserstoff zur Rückgewinnung von Chlor für die Synthese von Phosgen als Ausgangsprodukt für die Isocyanatherstellung.

Im ersten Schritt (a) des erfindungsgemäßen Verfahrens erfolgt die Herstellung von Phosgen durch Umsetzung von Chlor mit Kohlenmonoxid. Die Synthese von Phosgen ist hinlänglich bekannt und ist z.B. in Ullmanns Enzyklopädie der industriellen Chemie, 3. Auflage, Band 13, Seite 494-500 dargestellt. Weitere Verfahren zur Herstellung von Isocyanaten sind z.B. in US 4764308 und WO 03/072237 beschrieben. Im technischen Maßstab wird Phosgen überwiegend durch Umsetzung von Kohlenmonoxid mit Chlor bevorzugt an Aktivkohle als Katalysator hergestellt. Die stark exotherme Gasphasenreaktion erfolgt bei Temperaturen von mindestens 250°C bis maximal 600°C in der Regel in Rohrbündelreaktoren. Die Abführung der Reaktionswärme kann auf unterschiedliche Weise erfolgen, beispielsweise durch ein flüssiges Wärmetauschmittel, wie z.B. in WO 03/072237 beschrieben, oder durch Siedekühlung über einen Sekundärkühlkreislauf unter gleichzeitiger Nutzung der Reaktionswärme zur Dampferzeugung, wie z.B. in US 4764308 offenbart.

Aus dem gemäß Schritt (a) gebildeten Phosgen wird durch Umsetzung mit wenigstens einem organischen Amin oder einem Gemisch aus zwei oder mehreren Aminen in einem nächsten Verfahrensschritt (b) wenigstens ein Isocyanat gebildet. Der Verfahrensschritt (b) wird nachfolgend auch als Phosgenierung bezeichnet. Die Umsetzung erfolgt unter Bildung von Chlorwasserstoff als Nebenprodukt.

Die Synthese von Isocyanaten ist ebenfalls aus dem Stand der Technik hinlänglich bekannt, wobei in der Regel Phosgen in einem stöchiometrischen Überschuss, bezogen auf das Amin, eingesetzt wird. Üblicherweise findet die Phosgenierung gemäß (b) in der Flüssigphase statt, wobei das Phosgen und das Amin in einem Lösemittel gelöst sein können. Bevorzugte Lösemittel sind chlorierte aromatische Kohlenwasserstoffe, wie beispielsweise Chlorbenzol, o-Dichlorbenzol, p-Dichlorbenzol, Trichlorbenzole, die entsprechenden Chlortoluole oder Chlorxylole, Chlorethylbenzol, Monochlordiphenyl, α- bzw. β-Naphthylchlorid, Benzoesäureethylester, Phthalsäuredialkylester, Diisodiethylphthalat, Toluol und Xylole. Weitere Beispiele für geeignete Lösemittel sind aus dem Stand der Technik bekannt. Wie außerdem aus dem Stand der Technik, z.B. WO 96/16028, bekannt, kann als Lösemittel für Phosgen ebenso das gebildete Isocyanat selbst fungieren. In einer anderen, bevorzugten Ausführungsform findet die Phosgenierung, insbesondere geeigneter aromatischer und aliphatischer Diamine, in der Gasphase, d.h. oberhalb des Siedepunktes des Amins, statt. Die Gasphasenphosgenierung ist z.B. in EP 570 799 A beschrieben. Vorteile dieses Verfahrens gegenüber der ansonsten üblichen Flüssigphasenphosgenierung liegen in der Energieeinsparung, bedingt durch die Minimierung eines aufwändigen Lösemittel- und Phosgenkreislaufs.

Als organische Amine eignen sich prinzipiell alle primären Amine mit einer oder mehreren primären Aminogruppen, die mit Phosgen unter Bildung einer oder mehrerer Isocyanate mit einer oder mehreren Isocyanatgruppen reagieren können. Die Amine weisen mindestens eine, bevorzugt zwei, oder gegebenenfalls drei und mehr primäre Aminogruppen auf. So kommen als organische primäre Amine aliphatische, cycloaliphatische, aliphatisch-aromatische, aromatische Amine, Di- und/oder Polyamine in Frage, wie Anilin, Halogen-substituierte Phenylamine, z.B. 4-Chlorphenylamin, 1,6-Diaminohexan, 1-Amino-3,3,5-trimethyl-5-amino-cyclohexan, 2,4-, 2,6-Diaminotoluol oder deren Gemische, 4,4'-, 2,4'- oder 2,2'-Diphenylmethandiamin oder deren Gemische, wie auch höhermolekulare isomere, oligomere oder polymere Derivate der genannten Amine und Polyamine. Weitere mögliche Amine sind aus dem Stand der Technik bekannt. Bevorzugte Amine für die vorliegende Erfindung sind die Amine der Diphenylmethandiamin-Reihe (monomere, oligomere und polymere Amine), 2,4-, 2,6-Diaminotoluol, Isophorondiamin und Hexamethylendiamin. Bei der Phosgenierung erhält man die entsprechenden Isocyanate Diisocyanatodiphenylmethan (MDI, monomere, oligomere und polymere Derivate), Toluylendiisocyanat (TDI), Hexamethylendiisocyanat (HDI) und Isophorondiisocyanat (IPDI).

Die Amine können mit Phosgen in einer einstufigen oder zweistufigen oder ggf. mehrstufigen Reaktion umgesetzt werden. Dabei ist eine kontinuierliche wie auch diskontinuierliche Betriebsweise möglich.

Wird eine einstufige Phosgenierung in der Gasphase gewählt, so erfolgt die Umsetzung oberhalb der Siedetemperatur des Amins bevorzugt innerhalb einer mittleren Kontaktzeit von 0,5 bis 5 Sekunden und bei Temperaturen von 200 bis 600°C.

Bei der Phosgenierung in der Flüssigphase werden üblicherweise Temperaturen von 20 bis 240°C und Drücke von 1 bis ca 50 bar eingesetzt. Die Phosgenierung in der Flüssigphase kann einstufig oder mehrstufig durchgeführt werden, wobei Phosgen im stöchiometrischen Überschuss eingesetzt werden kann. Dabei werden die Aminlösung und die Phosgenlösung über ein statisches Mischelement vereinigt und anschließend beispielsweise von unten nach oben durch einen oder mehrere Reaktionstürme geführt, wo das Gemisch zum gewünschten Isocyanat ausreagiert. Neben Reaktionstürmen, die mit geeigneten Mischelementen versehen sind, können auch Reaktionsbehälter mit Rührvorrichtung eingesetzt werden. Außer statischen Mischelementen können auch spezielle dynamische Mischelemente Anwendung finden. Geeignete statische und dynamische Mischelemente sind aus dem Stand der Technik bekannt.

In der Regel wird die kontinuierliche Flüssigphasen-Isocyanatherstellung im industriellen Maßstab zweistufig durchgeführt. Dabei wird in der ersten Stufe im Allgemeinen bei Temperaturen von maximal 220°C, bevorzugt maximal 160°C aus Amin und Phosgen das Carbamoylchlorid sowie aus Amin und abgespaltenem Chlorwasserstoff Aminhydrochlorid gebildet. Diese erste Stufe ist stark exotherm. In der zweiten Stufe wird sowohl das Carbamoylchlorid zu Isocyanat und Chlorwasserstoff gespalten als auch das Aminhydrochlorid zum Carbamoylchlorid umgesetzt. Die zweite Stufe wird in der Regel bei Temperaturen von mindestens 90°C, vorzugsweise von 100 bis 240°C, durchgeführt.

Nach der Phosgenierung gemäß Schritt (b) erfolgt erfindungsgemäß in Schritt (c) die Abtrennung der bei der Phosgenierung gebildeten Isocyanate. Dies geschieht dadurch, dass zunächst das Reaktionsgemisch der Phosgenierung in einen flüssigen und einen gasförmigen Produktstrom in einer dem Fachmann bekannten Weise aufgetrennt wird. Der flüssige Produktstrom enthält im Wesentlichen das Isocyanat bzw. Isocyanatgemisch, das Lösemittel sowie einen geringen Teil an nicht umgesetztem Phosgen. Der gasförmige Produktstrom besteht im Wesentlichen aus Chlorwasserstoffgas, stöchiometrisch überschüssigem Phosgen, sowie geringfügigen Mengen an Lösemittel und Inertgasen, wie zum Beispiel Stickstoff und Kohlenmonoxid. Ferner wird der Flüssigstrom gemäß Schritt (c) anschließend einer Aufarbeitung zugeführt, vorzugsweise einer destillativen Aufarbeitung, wobei nacheinander Phosgen sowie das Lösemittel abgetrennt werden. Gegebenenfalls erfolgt gemäß Schritt (c) außerdem eine weitere Aufarbeitung der gebildeten Isocyanate. Dies geschieht beispielsweise, indem das erhaltene Isocyanatprodukt in einer dem Fachmann bekannten Weise fraktioniert wird.

Der bei der Reaktion von Phosgen mit einem organischen Amin erhaltene Chlorwasserstoff enthält im Allgemeinen organische Bestandteile, welche bei der elektrochemischen Oxidation einer wässrigen Chlorwasserstofflösung gemäß Schritt (g) stören können. Zu diesen organischen Bestandteilen zählen beispielsweise die bei der Isocyanatherstellung eingesetzten Lösungsmittel wie Chlorbenzol, o-Dichlorbenzol oder p-Dichlorbenzol. Erfolgt die Elektrolyse nach dem Membranverfahren, so könnte die Ionenaustauschermembran durch diese organischen Bestandteile oder durch anorganische Verunreinigungen, wie Eisen-, Silizium- oder Aluminium-Verbindungen, in ihrer Funktion beeinträchtigt werden. Die Verunreinigungen können sich auch auf der Ionenaustauschermembran ablagern und dadurch die Spannung der Elektrolyse erhöhen. Wird bei der Elektrolyse eine Gasdiffusionselektrode als Kathode eingesetzt, so kann auch der Katalysator der Gasdiffusionselektrode durch die anorganischen oder organischen Verunreinigungen deaktiviert werden. Darüber hinaus können sich diese Verunreinigungen auf dem Stromkollektor ablagern und dadurch den Kontakt zwischen Gasdiffusionselektrode und Stromkollektor verschlechtern, was einen Spannungsanstieg zur Folge hat. Wird zur Elektrolyse der Salzsäure das Diaphragmaverfahren eingesetzt, so können sich die genannten organischen und anorganischen Bestandteile auf den Graphitelektroden und/oder dem Diaphragma ablagern und dadurch ebenfalls die Elektrolysespannung erhöhen.

Dementsprechend erfolgt in einem weiteren Verfahrensschritt (d) die Abtrennung des bei der Phosgenierung gemäß Schritt (b) erzeugten Chlorwasserstoffs aus dem gasförmigen Produktstrom. Der gasförmige Produktstrom der bei der Abtrennung des Isocyanats gemäß Schritt (c) erhalten wird, wird gemäß Schritt (d) so behandelt, dass das Phosgen wieder der Phosgenierung und der Chlorwasserstoff einer elektrochemischen Oxidation zugeführt werden kann.

Die Abtrennung des Chlorwasserstoffs gemäß Schritt (d) erfolgt zunächst, indem Phosgen aus dem gasförmigen Produktstrom abgetrennt wird. Die Abtrennung des Phosgens gelingt durch Verflüssigung von Phosgen, beispielsweise an einem oder mehreren in Reihe geschalteten Kondensatoren. Die Verflüssigung erfolgt vorzugsweise bei Temperaturen im Bereich von -15 bis -40° in Abhängigkeit des eingesetzten Lösemittels. Durch diese Tiefkühlung können außerdem Teile der Lösemittelreste aus dem gasförmigen Produktstrom entfernt werden.

Zusätzlich oder alternativ kann das Phosgen mit einem kalten Lösemittel oder Lösemittel-Phosgen-Gemisch aus dem Gasstrom in einer oder mehreren Stufen ausgewaschen werden. Als Lösemittel hierfür eignen sich beispielsweise die bereits in der Phosgenierung eingesetzten Lösemittel Chlorbenzol und o-Dichlorbenzol. Die Temperatur des Lösemittels oder Lösemittel-Phosgen-Gemischs hierfür liegt im Bereich von -15 bis -46°C.

Das aus dem gasförmigen Produktstrom abgetrennte Phosgen kann wieder der Phosgenierung gemäß Schritt (b) zugeführt werden. Der nach Abtrennung des Phosgens und eines Teils des Lösungsmittelrestes erhaltene Chlorwasserstoff kann neben den Inertgasen wie Stickstoff und Kohlenmonoxid noch 0,1 bis 1 Gew.% Lösungsmittel und 0,1 bis 2 Gew.% Phosgen enthalten.

Gegebenenfalls erfolgt anschließend gemäß Schritt (d) eine Reinigung des Chlorwasserstoffs, um den Anteil an Lösungsmittel zu verringern. Dies kann beispielsweise mittels Ausfrieren erfolgen, indem in Abhängigkeit von den physikalischen Eigenschaften des Lösemittels der Chlorwasserstoff zum Beispiel durch eine oder mehrere Kühlfallen geleitet wird.

In einer bevorzugten Ausführungsform der gemäß Schritt (d) gegebenenfalls vorgesehenen Reinigung des Chlorwasserstoffs werden zwei in Serie geschaltete Wärmetauscher von dem Chlorwasserstoffstrom durchströmt, wobei das abzutrennende Lösemittel in Abhängigkeit des Festpunktes zum Beispiel bei -40°C ausgefroren wird. Die Wärmetauscher werden wechselweise betrieben, wobei im jeweils zuerst durchströmten Wärmetauscher der Gasstrom das zuvor ausgefrorene Lösemittel auftaut. Das Lösemittel kann wieder für die Herstellung einer Phosgenlösung eingesetzt werden. Im nachgeschalteten zweiten Wärmetauscher, der mit einem üblichen Wärmeträgermedium für Kältemaschinen, z.B. eine Verbindung aus der Reihe der Frigene, beaufschlagt ist, wird das Gas unter den Festpunkt des Lösemittels abgekühlt, sodass dieses auskristallisiert. Nach abgeschlossenem Auftau- und Kristallisationsvorgang werden der Gasstrom und der Kühlmittelstrom umgeschaltet, sodass sich die Funktion der Wärmetauscher umkehrt. Der chlorwasserstoffhaltige Gasstrom kann auf diese Weise auf vorzugsweise maximal 500 ppm, besonders bevorzugt maximal 50 ppm, ganz besonders bevorzugt auf maximal 20 ppm Lösemittelgehalt abgereichert werden.

Alternativ kann die Reinigung des Chlorwasserstoffs in zwei in Serie geschaltete Wärmetauscher gemäß US 6 719 957 erfolgen. Dabei wird der Chlorwasserstoff auf einen Druck von 5 bis 20 bar, bevorzugt 10 bis 15 bar, verdichtet und der komprimierte gasförmige Chlorwasserstoff mit einer Temperatur von 20 bis 60°C, bevorzugt 30 bis 50°C, einem ersten Wärmeaustauscher zugeführt. In diesem wird der Chlorwasserstoff mit einem kalten Chlorwasserstoff einer Temperatur von -10 bis -30°C, der aus einem zweiten Wärmetauscher stammt, gekühlt. Dabei kondensieren organische Bestandteile, die einer Entsorgung oder Wiederverwertung zugeführt werden können. Der in den ersten Wärmetauscher geleitete Chlorwasserstoff verlässt diesen mit einer Temperatur von -20 bis 0°C und wird in dem zweiten Wärmetauscher auf eine Temperatur von - 10 bis -30°C gekühlt. Das im zweiten Wärmetauscher anfallende Kondensat besteht aus weiteren organischen Bestandteilen sowie geringen Mengen Chlorwasserstoff. Zur Vermeidung eines Chlorwasserstoffverlustes wird das aus dem zweiten Wärmetauscher ablaufende Kondensat einer Abtrenn- und Verdampfereinheit zugeführt. Dies kann beispielsweise eine Destillationskolonne sein, in der aus dem Kondensat der Chlorwasserstoff ausgetrieben und in den zweiten Wärmetauscher zurückgeführt wird. Es ist auch möglich, den ausgetriebenen Chlorwasserstoff in den ersten Wärmetauscher zurückzuführen. Der in dem zweiten Wärmetauscher abgekühlte und von organischen Bestandteilen befreite Chlorwasserstoff wird mit einer Temperatur von -10 bis -30°C in den ersten Wärmetauscher geleitet. Nach Erwärmung auf 10 bis 30°C verlässt der von organischen Bestandteilen befreite Chlorwasserstoff den ersten Wärmetauscher.

In einem alternativen Verfahren erfolgt die gemäß Schritt (d) gegebenenfalls vorgesehene Reinigung des Chlorwasserstoffs mittels Adsorption von organischen Verunreinigungen, wie Lösemittelreste, an Aktivkohle. Dabei wird beispielsweise der Chlorwasserstoff nach Entfernung von überschüssigem Phosgen bei einem Druck von 0 bis 5 bar, vorzugsweise von 0,2 und 2 bar, über oder durch eine Aktivkohleschüttung geleitet. Die Strömungsgeschwindigkeiten und Verweilzeiten werden dabei in einer dem Fachmann bekannten Weise dem Gehalt an Verunreinigungen angepasst. Die Adsorption von organischen Verunreinigungen ist ebenso an anderen geeigneten Adsorptionsmitteln möglich, so z.B. an Zeolithen.

In einem weiteren alternativen Verfahren kann für die gemäß Schritt (d) gegebenenfalls vorgesehene Reinigung des Chlorwasserstoffs eine Destillation des Chlorwasserstoffes vorgesehen sein. Diese erfolgt nach Kondensation des gasförmigen Chlorwasserstoffes. Bei der Destillation des kondensierten Chlorwasserstoffes wird der gereinigte Chlorwasserstoff als Kopfprodukt der Destillation entnommen, wobei die Destillation unter dem Fachmann bekannte, für eine solche Destillation übliche Bedingungen von Druck, Temperatur u.ä. erfolgt.

Aus dem gemäß Schritt (d) abgetrennten und ggf. gereinigten Chlorwasserstoff wird danach in Schritt (e) eine wässrige Chlorwasserstofflösung hergestellt. Hierfür wird der Chlorwasserstoff vorzugsweise einer adiabaten Chlorwasserstoffabsorption zugeführt, die in einer Absorptionskolonne unter Zugabe eines geeigneten Absorptionsmittels erfolgt. In einer bevorzugten Ausführungsform besteht das Absorptionsmittel aus einer wässrigen Chlorwasserstofflösung (Salzsäure) im Konzentrationsbereich bis 20 Gew.%, bevorzugt 16 bis 18 Gew.%. Alternativ kann auch eine Salzsäure geringerer Konzentration oder entsalztes Wasser oder ein Dampfkondensat eingesetzt werden. Die adiabate Absorption von Chlorwasserstoff in wässriger Salzsäure zur Herstellung von konzentrierter Salzsäure ist bereits aus dem Stand der Technik bekannt. Die Absorption erfolgt zum Beispiel durch Einleiten des Chlorwasserstoffstroms in den unteren Teil einer Absorptionskolonne, in dem die Absorptionskolonne mit Stoffaustauschelementen, wie zum Beispiel Siebböden oder Packungen, ausgerüstet ist. Auf den oberen Teil der Absorptionskolonne oberhalb der Stoffaustauschelemente wird das Absorptionsmittel aufgegeben. Das Chlorwasserstoffgas wird im Gegenstrom an den Stoffaustauschelementen im Absorptionsmittel absorbiert, d.h. gelöst.

Der am Kopf der Absorptionskolonne austretende Gasstrom, die Brüden, besteht im prozess-üblichen Temperaturbereich von 90 bis 120°C, bevorzugt von 105 bis 109°C, im Wesentlichen aus Wasserdampf. Daneben sind noch Chlorwasserstoff, Inertgase wie Stickstoff und Kohlenmonoxid sowie noch nicht mit Wasser abreagiertes Phosgen und Restmengen an Lösemittel enthalten. Zur Abtrennung kondensierbarer Anteile, wie Wasser, Salzsäure und Lösemittelreste und zur Abführung der Kondensationswärme wird der gasförmige Kopfstrom bevorzugt einer Kondensationseinheit zugeführt. Diese Kondensationseinheit kann zum Beispiel aus einem oder mehreren nacheinander geschalteten kühlwasserbetriebenen Rohrbündelwärmetauschern bestehen. Der flüssige Ablauf dieses Kondensationssystems wird anschließend vorzugsweise einem Abscheider zugeführt, um die auskondensierten Lösemittelanteile von der wässrigen Salzsäurephase abzutrennen. Bei diesem Abscheider handelt es sich vorzugsweise um einen statischen Phasentrenner. Durch entsprechende Trennelemente in diesem Abscheider kann die Trennung von organischer und wässriger Phase unterstützt werden. Die abgeschiedene organische Phase wird einer geeigneten Verwertung zugeführt. Die an Lösemittel abgereicherte Salzsäurephase kann auf den oberen Teil der Absorptionskolonne rückgeführt werden.

Die im unteren Bereich der Absorptionskolonne ablaufende wässrige Chlorwasserstofflösung (Salzsäure) kann bei Bedarf mit einem dafür geeigneten Kühlaggregat abgekühlt, ggf. gemäß Schritt (f) gereinigt und anschließend der elektrochemischen Oxidation gemäß Schritt (g) zugeführt werden. Diese im Allgemeinen ca. 24 bis 30 gew.%ige, bevorzugt 27 bis 30 gew.%ige Salzsäure, im Folgenden auch als aufkonzentrierte Salzsäure bezeichnet, enthält Lösemittelanteile von bevorzugt maximal 0,05 Gew.%, besonders bevorzugt maximal 0,005 Gew.%. Der Phosgengehalt der Salzsäure beträgt bevorzugt 0,1 bis 0,0001 Gew.%, kann jedoch auch weniger als 0,0001 Gew.% betragen.

Die wässrige Chlorwasserstofflösung wird ggf. in einem Schritt (f) einer Reinigung, insbesondere zur weiteren Verringerung des Lösungsmittelanteils und des Phosgengehalts, unterworfen. Dies kann mittels Strippung in einer Kolonne in einer dem Fachmann bekannten Weise geschehen, z.B. durch Einleiten der aufkonzentrierten Salzsäure in eine Packungskolonne, die entweder mit einem Umlaufverdampfer oder mit einer Dampfeinspeisung versehen ist. Während die Brüden der Stripperkolonne zurück in die Absorptionskolonne geführt werden können, kann der flüssige Austrag der Kolonne als gereinigte aufkonzentrierte Salzsäure, gegebenenfalls über einen Kühler, der Salzsäure-Elektrolyse gemäß Schritt (g) zugeführt werden. Anstatt die Strippung in einer separaten Stripperkolonne durchzuführen, kann sie auch in der Absorptionskolonne selbst durch Direkteinspeisung von Dampf, bevorzugt in den unter der Absorptionskolonne angeordneten Abtriebsteil, erfolgen. Anstelle der Strippung in der Absorptionskolonne kann auch durch partielles Destillieren mit Hilfe eines der Absorptionskolonne nachgeschalteten Wärmetauschers der Lösemittelanteil im Chlorwasserstoff reduziert werden.

Darüber hinaus wird die wässrige Chlorwasserstofflösung in dem ggf. vorgesehenen Schritt (f) einer Reinigung zur Entfernung von Eisen-, Aluminium- und/oder Siliziumverbindungen unterworfen. Bevorzugt erfolgt die Entfernung von Eisen-, Aluminium- und/oder Siliziumverbindungen mittels chelatbildender Ionenaustauscher. Derartige Ionenaustauscher sind kommerziell erhältlich.

So kann beispielsweise die Entfernung von Eisen-Verbindungen durch Ionenaustauscher vom Typ Amberjet 4400CI der Fa. Rohm&Haas oder Lewatit M500 der Fa. LANXESS erfolgen. Bevorzugt beträgt die Konzentration der Salzsäure zur Entfernung von Eisen mindestens 8 Gew.%.

Zur Entfernung eisenhaltiger Verbindungen kann auch eine Fällung als schwerlösliche Verbindungen und eine anschließende Filtration vorgesehen sein.

Nach Herstellung einer wässrigen Chlorwasserstofflösung gemäß Schritt (e) sowie ggf. nach einer Reinigung der wässrigen Chlorwasserstofflösung gemäß Schritt (f) wird die Salzsäure einer Elektrolysezelle zugeführt. Die elektrochemische Oxidation der Salzsäure gemäß Schritt (g) kann nach dem Membranverfahren oder nach dem Diaphragmaverfahren in einer Zweiraum-Elektrolysezelle, bestehend aus Anodenraum und Kathodenraum, oder in einer Dreiraum-Elektrolyszelle, bestehend aus Anodenraum, Kathodenraum und einem Elektrolytraum zwischen Anoden- und Kathodenraum, durchgeführt werden. Bevorzugt wird eine Zweiraum-Elektrolysezelle gewählt. Beim Membranverfahren ist der Anodenraum von dem Kathodenraum durch eine Ionenaustauschermembran (nachfolgend vereinfacht auch als Membran bezeichnet), insbesondere eine Kationenaustauscher-membran, getrennt, beim Diaphragmaverfahren durch ein Diaphragma. Der Abstand der Elektroden (Anode und Kathode) von dem Diaphragma bzw. der Membran beträgt vorzugsweise 0 bis 3 mm, besonders bevorzugt 0 bis 2 mm. Geeignete Ionenaustauschermembrane sind kommerziell erhältlich, wie z.B. einschichtige Ionenaustauschermembrane mit Sulfonsäuregruppen. Beispielsweise kann eine Membran vom der Fa. DuPont Typ Nafion® 117 eingesetzt werden.

Als Diaphragma kann z.B. ein gewebtes Diaphragma gemäß DE 3 321 159 A eingesetzt werden. Hierfür können Fäden aus Kunststoff verwendet werden. So können beispielsweise Gewebe aus Polyvinylchlorid (PVC) oder Polyvinylidenfluorid (PVDF) oder auch aus Mischgeweben mit Fäden aus PVC und PVDF eingesetzt werden. Kett- oder Schussfäden können dabei neben monofilen aus multifilen Fäden, wie in DE 3 321 159 A beschrieben, bestehen. Nach dem Weben des Diaphragmas kann das Gewebe zwecks Optimierung der Gasdurchlässigkeit, z.B. durch Kalandrieren, verdichtet werden.

Bei der Elektrolyse von Salzsäure nach dem Diaphragmaverfahren oder dem Membranverfahren können Elektroden eingesetzt werden, die Graphit enthalten, wobei die Anode und/oder die Kathode bevorzugt im Wesentlichen aus Graphit bestehen. Besonders bevorzugt werden bipolare Elektroden aus Graphit eingesetzt. Eine besonders vorteilhafte Ausführung der Kathoden besteht gemäß DE 4 417 744 A darin, dass besonders die Kathoden aber auch die Anoden aus Graphit mit einer edelmetallhaltigen Beschichtung, zum Beispiel Iridium, versehen sind.

Die Graphit-Anoden weisen insbesondere eine geometrische Form auf, wie sie aus DE 3 041 897 A bekannt ist. Die Kathoden weisen vorzugsweise eine ähnliche Struktur wie die Anoden auf. Die Form der Anode und/oder Kathode weist bevorzugt vertikal angeordnete Nuten, Rillen, Kerben, Vertiefungen oder dergleichen auf. Diese Nuten dienen im Wesentlichen dazu, das sich bei der Elektrolyse bildende Gas, d.h. Chlor und Wasserstoff, aus dem engen Spalt zwischen der jeweiligen Elektrode und dem Diaphragma bzw. der Membran nach oben abzuleiten. Besonders bevorzugt haben die Nuten eine Tiefe von 5 bis 35 mm, ganz besonders bevorzugt von 15 bis 25 mm und eine Breite von bevorzugt 1 bis 5 mm. Zwischen zwei benachbarten, im Wesentlichen parallel zueinander angeordneten Nuten beträgt der Abstand 4 bis 6 mm. In einer weiteren möglichen Ausführungsform der Nuten variieren die Tiefe und/oder die Breite der Nuten entlang ihrer Länge. So kann die Tiefe der Nuten von 12 bis 15 mm am unteren Ende der Nuten und 20 bis 30 mm am oberen Ende der Nuten betragen.

Die Salzsäure wird als Elektrolyt sowohl im Anodenraum als auch im Kathodenraum eingesetzt. Bei der Elektrolyse wird an der Anode Chlor, an der Kathode Wasserstoff erzeugt.

Eine bevorzugte Betriebsweise der elektrochemischen Oxidation von Salzsäure besteht darin, dass der Salzsäure, welche als Elektrolyt im Kathodenraum dient, Metallionen aus der Gruppe der Platinmetalle, vorzugsweise Platin und/oder Palladium, zugesetzt werden. So können beispielsweise Lösungen der Hexachloroplatinat (IV)-Säure (H₂PtCl₆) oder Lösungen des Dinatriumtetrachloropalladas (II) (Na₂PdCl₄) oder auch deren Mischungen zugegeben werden. Die Zugabe kann kontinuierlich oder diskontinuierlich erfolgen. Die Zugabe von Metallionen zur Salzsäure im Kathodenraum dient der Aufrechterhaltung einer niedrigen Elektrolysespannung im Bereich von 1,6 bis 2,1 V, verglichen mit 2,2 bis 2,3 V ohne Zusatz von Metallionen, bei 5 kA/m² und 70 bis 80°C und eine bevorzugt 15 bis 25%, besonders bevorzugt ca. 20%ige, Salzsäure. Demnach wird eine Menge an Metallionen zugegeben, die ausreicht, um die Elektrolysespannung im Bereich von 1,8 bis 2,1 zu halten. Dies bedeutet, dass bei steigender Elektrolysespannung während des Betriebes die Zugabe an Metallionen erhöht wird.

Die Elektrolyse gemäß Schritt (g) wird bevorzugt bei einer Temperatur von 50 bis 90°C durchgeführt. Die Konzentration der eingesetzten wässrigen Lösung von Chlorwasserstoff beträgt vorzugsweise 15 bis 25 Gew.%. Die Elektrolyse kann bei einem Absolutdruck von 1 bar oder auch bei höherem Druck von bis zu 2 bar durchgeführt werden. Höhere Drücke sind generell möglich, erfordern jedoch einen entsprechend höheren Aufwand bei der Konstruktion der Elektrolysezelle. Der Differenzdruck zwischen Anodenkammer und Kathodenkammer beträgt bevorzugt 0 bis 10 mbar, besonders bevorzugt ca. 1 mbar, so dass aufgrund des höheren Drucks auf der Anodenseite Spuren des gebildeten Chlorgases über das Diaphragma auf die Kathodenseite gelangt und somit sich mit dem kathodisch gebildeten Wasserstoff vermischen kann.

In einer alternativen Ausführungsform erfolgt die elektrochemische Oxidation der wässrigen Lösung von Chlorwasserstoff gemäß Schritt (g) nach dem Membranverfahren mit einer Gasdiffusionselektrode als Kathode. Dabei kann die Elektrolysezelle sowohl aus zwei Kammern als auch aus drei Kammern, bevorzugt jedoch aus zwei Kammern, bestehen. Der Kathodenhalbzelle wird ein sauerstoffhaltiges Gas, z.B. Sauerstoff, Luft oder mit Sauerstoff angereicherte Luft, zugeführt. Der Sauerstoff wird an der Gasdiffusionselektrode reduziert, wobei Wasser gebildet wird. Der Anodenhalbzelle wird die wässrige Chlorwasserstoff-Lösung zugeführt, wobei der Chlorwasserstoff an der Anode zu Chlor oxidiert wird. Die Anodenhalbzelle und die Kathodenhalbzelle sind durch eine Kationenaustauschermembran voneinander getrennt. Die Elektrolyse von Salzsäure unter Verwendung einer Gasdiffusionselektrode als Kathode ist z.B. in WO 00/73538 beschrieben.

Die Elektrolysezelle kann entweder aus einem nichtmetallischen Werkstoff gemäß DE 103 47 703 A oder aus einem metallischen Werkstoff bestehen. Als metallischer Werkstoff für die Elektrolysezelle eignet sich z.B. Titan oder eine Titan-Legierung, wie eine Titan-Palladium-Legierung. Dabei sind die Halbschalen für die Anode- und Kathodenhalbzelle, der Stromverteiler und die Stromzuführungen aus Titan oder einer Titan-Legierung gefertigt.

Die Anode kann z.B. gemäß DE 102 34 806 A ausgeführt sein. Dabei besteht die Anode aus Metall, vorzugsweise aus Titan mit einer Beschichtung aus Edelmetalloxid, z.B. aus Rutheniumoxid. Weiterhin kann die Anode aus Titan gemäß DE 102 00 072 A eine Zwischenschicht aus Titancarbid oder Titanborid aufweisen, welche mittels Plasma- oder Flammspritzen auf die Titananode aufgebracht wird, bevor die Beschichtung aus einem Edelmetalloxid aufgebracht wird. Gemäß DE 102 34 806 A weist die Metallanode Öffnungen für den Durchlass des während der Elektrolyse gebildeten Gases auf, wobei die Öffnungen bevorzugt Leitstrukturen aufweisen, welche das gebildete Gas auf die der Ionenaustauschermembran abgewandten Seite der Metallanode ableiten. Dabei sollte die Gesamtquerschnittsfläche der Öffnungen im Bereich von 20% bis 70% der Fläche betragen, welche durch die Höhe und Breite der Anode gebildet wird. Die Metallanode kann außerdem einen wellenförmigen, zickzackförmigen oder rechteckförmigen Querschnitt aufweisen. Die Tiefe der Anode sollte dabei mindestens 1 mm betragen. Das Verhältnis von elektrochemisch aktiver Fläche der Metallanode zu der Fläche, welche durch die Höhe und Breite der Metallelektrode gebildet wird, sollte mindestens 1,2 betragen. In einer speziellen Ausführungsform kann die Metallanode aus zwei aneinander liegenden Streckmetallen bestehen, wobei das zur Ionenaustauschermembran weisende Streckmetall feiner strukturiert ist als das von der Ionenaustauschermembran abweisende Streckmetall. Dabei ist ferner das feiner strukturierte Streckmetall flach gewalzt und das gröber strukturierte Streckmetall so angeordnet, dass die Maschenstege in Richtung der Kathode geneigt sind und als Leitstrukturen dienen. Alternativ kann die Anode auch aus einem Streckmetall bestehen. Grundsätzlich sollte die Anode eine freie Fläche von 15 bis 70 % aufweisen. Die Dicke der Streckmetalle ist so zu wählen, dass kein zusätzlicher elektrischer Widerstand bei einer bipolaren Verschaltung der einzelnen Elektrolysezellen (Zellelemente) zu einem Elektrolyseur auftritt. Der elektrische Widerstand hängt im Wesentlichen von der elektrischen Kontaktierung der Anode ab, wie zum Beispiel Anzahl der stromzuführenden Verbindungselemente zwischen Anode und Rückwand der Anodenhalbzelle.

Bei der Elektrolyse mittels Gasdiffusionselektrode können der Anodenraum und der Kathodenraum von einer handelsüblichen Ionenaustauschermembran getrennt sein. Beispielsweise können lonenaustauschermembranen der Fa. DuPont vom Typ Nafion® 324 oder Nafion® 117 eingesetzt werden. Bevorzugt wird eine Membran eingesetzt, welche, wie in WO 05/12596 beschrieben, auf der zur Gasdiffusionselektrode gewandten Seite eine glatte Oberflächenstruktur aufweist. Die glatte Oberflächenstruktur der Membran erlaubt es, dass die Gasdiffusionselektrode und die Membran derart aneinander anliegen, dass unter einem Druck von 250 g/cm² und einer Temperatur von 60 °C die Kontaktfläche wenigstens 50 % der geometrischen Fläche der Membran beträgt.

Der kathodische Stromverteiler, auf den die Gasdiffusionselektrode aufgebracht wird, ist bevorzugt gemäß DE 102 03 689 A ausgeführt. Dieser weist eine freie Fläche von weniger als 65 %, jedoch von mehr als 5 % auf. Die Dicke des Stromverteilers beträgt mindestens 0,3 mm. Er kann aus einem Streckmetall, Netz, Gewebe, Schaum, Vlies, Schlitzblech oder Lochplatte aus Metall bestehen. Vorzugsweise besteht der kathodische Stromverteiler aus einem Streckmetall mit einer Maschenlänge von 4 bis 8 mm, einer Maschenbreite 3 bis 5 mm, einer Stegbreite von 0,4 bis 1,8 mm und einer Dicke von 0,4 bis 2 mm. Zusätzlich kann der kathodische Stromverteiler ein zweites Streckmetall als Träger für das erste Streckmetall aufweisen. Das zweite Streckmetall als Träger hat bevorzugt eine Maschenlänge von 10 bis 40 mm, eine Maschenbreite von 5 bis 15 mm, eine Stegbreite von 2 bis 5 mm sowie eine Dicke von 0,8 bis 4 mm. Als Träger kann auch ein Netz eingesetzt werden, welches vorzugsweise eine Drahtdicke von 1 bis 4 mm und eine Maschenweite von 7 bis 25 mm besitzt. Weiterhin kann als Träger ein Lochblech oder Schlitzblech eingesetzt werden, welches bevorzugt eine offene Fläche von weniger als 60 % und eine Dicke von 1 bis 4 mm besitzt. Als Werkstoff für den kathodischen Stromverteiler kann z.B. Titan oder eine edelmetallhaltige Titanlegierung, wie z.B. Titan-Palladium eingesetzt werden. Ist der Stromverteiler ein Streckmetall, so ist dieses bevorzugt gewalzt.

Als Gasdiffusionselektrode kann eine handelsübliche Gasdiffusionselektrode eingesetzt werden, die mit einem geeigneten Katalysator ausgerüstet ist. Geeignete Katalysatoren enthalten gemäß WO 00/73538 Rhodium und/oder wenigstens ein Rhodiumsulfid oder eine Mischung aus Rhodium und wenigstens einem Rhodiumsulfid. Gemäß EP 931 857 A können außerdem Rhodium und/oder Rhodiumoxid oder deren Mischungen eingesetzt werden. Die Gasdiffusionselektrode besteht bevorzugt aus einem elektrisch leitfähigen Gewebe, Papier oder Vlies aus Kohlenstoff, wobei das Gewebe, Papier oder Vlies auf einer Seite mit einer kohlenstoffhaltigen Katalysatorschicht und auf der anderen Seite mit einer Gasdiffusionsschicht versehen ist. Der Katalysator wird bevorzugt auf einen Träger, vorzugsweise aus Kohlenstoff, aufgebracht, wobei Polytetrafluorethylen-Teilchen integriert sind, die an die Trägerstruktur gekoppelt sind. Die Gasdiffusionsschicht besteht vorzugsweise aus Kohlenstoff und Polytetrafluorethylen-Teilchen, bei der beispielsweise das Verhältnis von Kohlenstoff zu PTFE 50:50 beträgt. Die Gasdiffusionselektrode kann beispielsweise so angeordnet werden, dass sie nicht fest mit der Ionenaustauschermembran verbunden ist. Die Kontaktierung der Gasdiffusionselektrode mit dem Stromverteiler und der Ionenaustauschermembran erfolgt bevorzugt durch Presskontakt, d.h. die Gasdiffusionselektrode, der Stromverteiler und die Membran werden aneinander gepresst. Die Gasdiffusionselektrode kann mit dem Stromkollektor gemäß DE 101 48 600 A verbunden sein.

Die Elektrolyse von Salzsäure nach dem Membranverfahren mit Gasdiffusionselektrode wird üblicherweise bei einer Temperatur von 40 bis 70°C durchgeführt. Die Konzentration der wässrigen Lösung von Chlorwasserstoff im Anodenraum beträgt 10 bis 20 Gew.%, bevorzugt 12 bis 17 Gew.%. Die Zelle kann beispielsweise so betrieben werden, dass der Druck im Anodenraum höher ist als der Druck im Kathodenraum. Dadurch wird die Kationenaustauschermembran auf die Gasdiffusionselektrode und diese wiederum auf den Stromverteiler gedrückt. Alternativ kann eine Konstruktion der Elektrolysezelle gewählt werden, die in DE 101 38 214 A beschrieben wird. Die Anode und/oder der Stromverteiler sind elastisch gelagert, beispielsweise indem sie mittels Federn mit der Rückwand der jeweiligen Halbzelle verbunden sind. Beim Zusammenbau der Zelle entsteht eine sogenannte zero gap Anordnung, bei der die Anode in direktem Kontakt mit der Ionenaustauschermembran, diese wiederum in direktem Kontakt mit der Gasdiffusionselektrode und diese wiederum in direktem Kontakt mit dem Stromverteiler steht. Die elastische Lagerung bewirkt das Aneinanderpressen von Anode, Membran, Gasdiffusionselektrode und Stromverteiler.

In einer bevorzugten Ausführungsform des Elektrolyseverfahrens wird beim Anfahren der Elektrolysezelle gemäß DE 10 152 275 A das Anodenhalbelement wird mit einer 5 bis 20 gew.%igen Salzsäure gefüllt, wobei die Salzsäure mindestens 10 ppm freies Chlor enthält und die Konzentration der Salzsäure während der Inbetriebnahme mehr als 5 Gew.% beträgt. Der Volumenstrom der Salzsäure durch den Anodenraum wird so eingestellt, dass zu Beginn der Elektrolyse die Salzsäure mit einer Geschwindigkeit im Anodenraum von 0,05 bis 0,15 cm/s strömt. Die Elektrolyse wird mit einer Stromdichte von 0,5 bis 2 kA/m² gestartet und in Zeitintervallen von 5 bis 25 Minuten um jeweils 0,5 bis 1,5 kA/m² erhöht. Nachdem eine vorgegebene Stromdichte von vorzugsweise 4 bis 7 kA/m² erreicht ist, wird der Volumenstrom der Salzsäure so eingestellt, dass die Salzsäure im Anodenhalbelement mit einer Geschwindigkeit von 0,2 bis 0,4 cm/s strömt.

Eine besonders vorteilhafte Betriebsweise der Elektrolysezelle kann gemäß DE 101 38 215 A erfolgen, wonach die Elektrolysezelle zur Erniedrigung der Zellspannung mit einem erhöhten Druck in der Kathodenkammer betrieben wird. Der Differenzdruck zwischen Anodenraum und Kathodenraum sollte 0,01 bis 1000 mbar und der Sauerstoffdruck im Kathodenraum mindestens 1,05 bar absolut betragen.

Erfindungsgemäß wird im nächsten Verfahrensschritt (h) wenigstens ein Teil des gemäß Schritt (g) hergestellten Chlors in die Herstellung von Phosgen gemäß Schritt (a) rückgeführt. Vor der Rückführung wird das Chlor vorzugsweise in einer ein- oder mehrstufigen Kühlung mittels eines Kühlaggregats, z.B. eines Röhrenwärmetauscher, abgekühlt und getrocknet. Die Trocknung kann zum Beispiel mit Hilfe eines geeigneten Trocknungsmittels in einer mit Stoffaustauschelementen bestückten Absorptionskolonne erfolgen. Ein geeignetes Trocknungsmittel kann, wie z.B. in DE 10 235 476 A beschrieben, neben Molsieben oder hygroskopischen Adsorbentien z.B. Schwefelsäure sein. Die Trocknung kann ein- oder mehrstufig erfolgen. Bevorzugt erfolgt die Trocknung zweistufig, indem das zu trocknende Chlor in einer ersten Stufe mit einer Schwefelsäure geringerer Konzentration, vorzugsweise 70 bis 80%, besonders bevorzugt 75 bis 80%, in Kontakt gebracht wird. In einer zweiten Stufe wird die Restfeuchte mittels einer höher konzentrierten Schwefelsäure von bevorzugt 88 bis 96%, besonders bevorzugt 92-96%, aus dem Chlor entfernt. Das auf diese Art getrocknete Chlor mit einer Restfeuchte von bevorzugt maximal 100 ppm, besonders bevorzugt maximal 20 ppm kann durch einen Tröpfchenabscheider geleitet werden, um gegebenenfalls darin noch enthaltene Schwefelsäuretröpfchen zu entfernen.

Die Kreislauffahrweise des erfindungsgemäßen Verfahrens erfordert es, neben dem gemäß Schritt (g) mittels Elektrolyse hergestellten Chlors für die Phosgenherstellung gemäß Schritt (a) eine weitere Teilmenge Chlor bereitzustellen, da Verluste an Chlor und Chlorwasserstoff in dem Chlor-Chlorwasserstoff-Kreislauf auftreten. Die Bereitstellung einer weiteren Teilmenge Chlor kann in Form von elementarem Chlor aus einer externen Quelle, beispielsweise der Elektrolyse einer wässrigen Natriumchloridlösung, stammen. Die auftretenden Verluste von Chlor und Chlorwasserstoff können aber auch dadurch ausgeglichen werden, dass eine Teilmenge Chlorwasserstoff aus einer externen Quelle bereitgestellt wird. Eine Teilmenge Chlorwasserstoff in Form einer wässrigen Chlorwasserstofflösung aus einer externen Quelle, z.B. aus einem Produktionsverfahren, bei dem eine wässrige Chlorwasserstofflösung als Nebenprodukt anfällt, wird vorzugsweise als ca 30 Gew.%ige Salzsäure der Elektrolyse gemäß Schritt (g) zugeführt, in Schritt (e) zur Herstellung der wässrigen Chlorwasserstofflösung zugeführt Eine Salzsäure geringerer Konzentration kann alternativ der Absorption von Chlorwasserstoff gemäß Schritt (e) zugeführt werden.

Wird die Fehlmenge durch Chlor ersetzt, kann dieses Chlor, das beispielsweise durch Steinsalzelektrolyse hergestellt wird, in geringen Mengen Brom oder Jod enthalten. Wird dieses Chlor für die Herstellung von MDI eingesetzt, kann es bei einer bestimmten Konzentration an Brom- und Jodverbindungen zu einer Verfärbung der aus MDI hergestellten Polyurethanprodukte kommen, wie z.B. in DE 10 235 476 A beschrieben. Das nach dem erfindungsgemäßen Verfahren rückgeführte Chlor hingegen ist weitgehend brom- und jodfrei, sodass sich ein gewisser Brom- und Jodanteil entsprechend dem Verhältnis des von außen zugeführten Chlors zu dem rückgeführten Chlor einstellt. Eine bevorzugte Ausführungsform des erfindungsgemäßen Verfahrens besteht demnach darin, dass die von außen zugeführte weitere Teilmenge Chlor in der Herstellung von Phosgen für die TDA-Phosgenierung eingesetzt wird, während das brom- und jodarme Chlor aus der Elektrolyse gemäß Schritt (g) in der Herstellung von Phosgen für die Phosgenierung von MDA (Diphenylmethandiamin) genutzt wird. Brom und Jod werden bei der Herstellung von TDI durch Phosgenierung von TDA im TDI gebunden und damit dem Chlorwasserstoff-Kreislauf entzogen. Bei der destillativen Aufarbeitung von TDI werden Brom und Jod jedoch vom TDI getrennt und verbleiben im Rückstand.

In einer weiteren bevorzugten Ausführungsform des erfindungsgemäßen Verfahrens wird das bei der Herstellung von Phosgen gemäß Schritt (a) eingesetzte Kohlenmonoxid durch Umsetzung von Methan mit Wasser in einem Steamreformer hergestellt und der dabei anfallende Wasserstoff mit wenigstens einer organischen Nitroverbindung zu wenigstens einem Amin umgesetzt, welches bei der Herstellung des Isocyanats gemäß Schritt (b) verwendet wird. Die Herstellung von Kohlenmonoxid durch Umsetzung von Methan mit Wasser in einem Steamreformer, ist seit langem bekannt. Ebenfalls ist die Umsetzung von Wasserstoff mit einer organischen Dinitroverbindung zur Herstellung eines Amins (Hydrierung) bekannt. Bei Einsatz eines Steamreformers zur Herstellung von Kohlenmonoxid stehen die stöchiometrisch benötigte Menge an Kohlenmonoxid für die Phosgenherstellung und die stöchiometrische Menge an Wasserstoff für die Hydrierung der Dinitroverbindungen zur Verfügung. Als Nitroverbindungen können beispielsweise Nitrobenzol und Dinitrotoluol (DNT) eingesetzt werden. Nitrobenzol und Dinitrotoluol werden zu Anilin und Toluylendiamin (TDA) hydriert. Anilin wird zu Polyaminen der Diphenylmethanreihe weiterverarbeitet. Neben anderen Aminen können MDA und TDA zur Isocyanatherstellung gemäß Schritt (c) eingesetzt werden. In die Wirtschaftlichkeitsbetrachtung des Gesamtprozesses zur Herstellung von Isocyanaten wird auch die Herstellung von Kohlenmonoxid einbezogen, wobei bevorzugt das Kohlenmonoxid aus Erdgas in einem Steamreformer hergestellt wird. Werden andere Reformerprozesse angewandt, z.B. Kohlevergasung oder Cracken von Erdölfraktionen, so werden andere Verhältnisse von Kohlenmonoxid zu Wasserstoff erhalten. Je größer das Verhältnis von Kohlenmonoxid zu Wasserstoff ist, desto unwirtschaftlicher wird das Gesamtverfahren, da der fehlende Wasserstoff für die Hydrierung der Dinitroverbindung zu den homologen Diaminen aus einer weiteren Quelle zugeführt werden muss. Der fehlende Wasserstoff kann beispielsweise durch die Elektrolyse von Salzsäure nach dem Diaphragmaverfahren bereitgestellt werden.

Die Vorteile des erfindungsgemäßen integrierten Verfahrens zur Herstellung von Isocyanaten unter elektrochemischer Oxidation einer wässrigen Lösung des bei der Isocyanatherstellung anfallenden Chlorwasserstoffs zur Rückgewinnung von Chlor für die Synthese von Phosgen liegen darin, dass die elektrochemische Oxidation einfacher betrieben werden kann als eine katalytische Oxidation nach dem Deacon-Verfahren. Der einfachere Betrieb bezieht sich auf das An- und Abfahren der Elektrolysezellen sowie die Anpassung an variable Lastzustände sowie an eine höhere oder niedrigere Kapazität der Anlage. Außerdem kann die elektrochemische Oxidation einer wässrigen Lösung von Chlorwasserstoff einfacher betrieben werden als eine Gasphasenelektrolyse von Chlorwasserstoff, da nicht in der Gasphase, sondern mit einer Lösung, gearbeitet wird.

Die Herstellung von Isocyanaten im Verbund mit der elektrochemischen Oxidation von Salzsäure bietet durch die Erzeugung einer aufkonzentrierten Salzsäure von rund 30% aus einer Salzsäure von rund 17% in Schritt (f) zusätzlich die Möglichkeit, bei Bedarf aufkonzentrierte Salzsäure für andere Anwendungen dem Kreislauf zu entnehmen. Eine mögliche Verwendung dieser aufkonzentrierten Salzsäure liegt im Nahrungsmittelbereich. Hierfür kann für die nach dem erfindungsgemäßen Verfahren hergestellte aufkonzentrierte Salzsäure z.B. durch absorptive Nachreinigung an einem Aktivkohlebett, wie sie aus dem Stand der Technik bekannt ist, eine für die Nahrungsmittelindustrie ausreichend hohe Reinheit erreicht werden. Eine weitere Möglichkeit besteht darin, die aufkonzentrierte Salzsäure als Katalysator bei der Herstellung von MDA einzusetzen. Üblicherweise erfolgt die Herstellung von Polyaminen der Diphenylmethanreihe durch Umsetzung von Anilin und Formaldehyd in Gegenwart saurer Katalysatoren, wie aus dem Stand der Technik hinlänglich bekannt. Als saurer Katalysator wird dabei üblicherweise Salzsäure eingesetzt.

### Beispiel

Das Beispiel geht aus von dem noch Phosgen enthaltenden Chlorwasserstoffgas (HCl) aus einer MDI-Produktion, das nach Abtrennung des MDI erhalten wird.

Nach der Entfernung von Phosgen aus dem HCl-Gas werden 44.660 kg/h HCl auf 13,4 bar verdichtet, die Temperatur der HCl beträgt ca. 40°C, der Gehalt an Monochlorbenzol (MCB) beträgt 200 ppm, und der Gehalt an Orthodichlorbenzol (ODB) 70 ppm. Dieser HCl-Strom wird einem ersten Wärmetauscher zugeführt und auf ca. -9,2°C abgekühlt. Hierbei kondensiert ein Teil des Chlorwasserstoffes zusammen mit ODB und MDB aus. Dieser Anteil beträgt ca. 62 kg/h, die Temperatur beträgt -9,2°C und wird einer Separator-Verdampfungseinheit zugeführt. Der gereinigte Reststrom von 44.598 kg/h wird einem zweiten Wärmetauscher 2 zugeführt und dort auf minus 23,4°C abgekühlt. Hierbei kondensiert wiederum ein Teil des Chlorwasserstoffes mit den Verunreinigungen MCB, ODB und eventuellen anderen Hochsiedern aus. Dieser Teilstrom beträgt 998 kg/h und wird der Separator-Verdampfungseinheit zugeführt. Der verbleibende zweifach gereinigte HCl-Reststrom von 44.573 kg/h und -23,4°C wird zur Kühlung des oben genannten Roh-HCl-Gasstromes auf den ersten Wärmetauscher zurückgeführt, dort auf ca. 21°C erwärmt und anschließend der HCl-Absorption in Wasser zugeführt. Der aus dem ersten Wärmetauscher austretende, erwärmte HCl-Strom hat einen MCB- und ODB-Gehalt von weniger als 1 ppm.

Die HCl-Teilströme (62 kg/h und 998 kg/h) aus dem ersten und zweiten Wärmetauscher werden einer Separator-Verdampfungseinheit zur Abtrennung von Chlorwasserstoff von den Verunreinigungen (Hochsiedern) zugeführt. Aus der Separator-Verdampfungseinheit werden 87 kg/h HCl mit einer Temperatur von ca. minus 12,2°C ausgeschleust, der Rest von 973 kg/h dem zweiten Wärmetauscher zugeführt. Die Separator-Verdampfungseinheit kann beispielsweise eine Destillationskolonne mit einem Verdampfer am Kolonnenboden sein.

Wie beschrieben wird der zweifach gereinigte Chlorwasserstoff in Wasser absorbiert, wobei eine 30 %ige Salzsäure entsteht.

Die 30 %ige Salzsäure wird einer Salzsäure-Elektrolyse, bei der als Kathode eine Sauerstoffverzehrkathode eingesetzt wird, zugeführt. Anoden- und Kathodenraum der Elektrolyse werden von einer Ionenaustauscher-Membran der Fa. DUPONT Nafion 324 getrennt. Die Temperatur des Anolyten beträgt 50°C, die Stromdichte 5 kA/m² bei einer Elektrolysespannung von 1,39 V. Die Anode besteht aus Titan, dass mit einer Edelmetallbeschichtung der Fa. DENORA ausgerüstet ist. Als Sauerstoffverzehrkathode wird eine Rhodiumsulfid-haltige Gasdiffusionselektrode der Fa. ETEK eingesetzt, die auf einem Stromverteiler aus Titan, welches Palladium-stabilisiert ist, aufliegt. Der Differenzdruck zwischen Anodenraum und Kathodenraum wird so eingestellt, dass die Membran auf die Sauerstoffverzehrkathode und den Stromverteiler gedrückt wird. Der Differenzdruck beträgt 200 mbar. Die Elektrolysezelle wird bei einem Absolutdruck von 1,01 bar betrieben. Die Anodenkammern der Elektrolyse werden mit 1466 t/h einer Salzsäure der Konzentration 14 Gew.% beschickt, den Anodenkammern wird eine Salzsäure der Konzentration 12,2 Gew.% entnommen. Kontinuierlich wird ein Purge-Strom von 96 t/h der 12,2 Gew.%igen Salzsäure abgenommen, der verbleibende Strom wird mit 148,5 t/h der gereinigten 30 %igen Salzsäure aufgestärkt und der Elektrolyse erneut zugeführt. Den Anodenkammern können 32,4 t/h Chlor entnommen werden.

Über die Membran erfolgt ein Wassertransport von 1,2 mol je mol Proton, so dass 19,7 t/h Wasser über die Membran in den Kathodenraum gelangen und dort mit dem Reaktionswasser aus der Sauerstoffreduktion als Kondensat abgeführt werden. Es werden 28,1 t/h Kondensat mit ca. 0,8 Gew.% HCl erhalten. Den Kathodenkammern wird 7,29 t/h reiner Sauerstoff zugeführt.

## Patentansprüche

1. Verfahren zur Herstellung von Isocyanaten, umfassend die folgenden Schritte:
(a) Herstellung von Phosgen durch Umsetzung von Chlor mit Kohlenmonoxid
(b) Umsetzung des gemäß Schritt (a) gebildeten Phosgens mit wenigstens einem organischen Amin unter Bildung wenigstens eines Isocyanats und von Chlorwasserstoff
(c) Abtrennung und Aufarbeitung der in Schritt (b) gebildeten Isocyanate
(d) Abtrennung des gemäß Schritt (b) gebildeten Chlorwasserstoffs
(e) Herstellung einer wässrigen Lösung des Chlorwasserstoffs
(f) Reinigung der wässrigen Lösung von Chlorwasserstoff durch Strippung mittels Wasserdampf, so dass der Anteil organischer Lösungsmittel maximal 0,05 Gew.-% beträgt,
(g) Elektrochemische Oxidation wenigstens eines Teils der wässrigen Chlorwasserstofflösung zu Chlor
(h) Rückführung wenigstens eines Teils des gemäß Schritt (g) hergestellten Chlors in die Herstellung von Phosgen gemäß Schritt (a).

2. Verfahren gemäß Anspruch 1, **dadurch gekennzeichnet, dass** die Abtrennung gemäß Schritt (d) des gemäß Schritt (b) gebildeten Chlorwasserstoffs eine Abtrennung von Phosgen mittels Verflüssigung umfasst.

3. Verfahren gemäß Anspruch 1 oder 2, **dadurch gekennzeichnet, dass** gemäß Schritt (d) eine Reinigung des Chlorwasserstoffs mittels Ausfrieren erfolgt.

4. Verfahren gemäß einem der Ansprüche 1 bis 3, **dadurch gekennzeichnet, dass** die Herstellung der wässrigen Chlorwasserstofflösung gemäß Schritt (e) durch Absorption in einer wässrigen Lösung von Chlorwasserstoff, bevorzugt mit einer Konzentration von 15 bis 20 Gew.%, erfolgt.

5. Verfahren gemäß einem der Ansprüche 1 bis 4, **dadurch gekennzeichnet, dass** gemäß Schritt (f) zusätzlich eine Entfernung von Eisen-, Silizium- und/oder Aluminium-Verbindungen aus der wässrigen Chlorwasserstofflösung mittels chelatbildender Ionenaustauscher erfolgt.

6. Verfahren gemäß Anspruch 5, **dadurch gekennzeichnet, dass** die wässrige Chlorwasserstofflösung eine mindestens 8 gew.%ige Lösung ist.

7. Verfahren gemäß einem der Ansprüche 1 bis 6, **dadurch gekennzeichnet, dass** die elektrochemische Oxidation der wässrigen Chlorwasserstoff-Lösung gemäß Schritt (g) in einer Elektrolysezelle erfolgt, in welcher der Anodenraum und der Kathodenraum durch eine Ionenaustauschermembran getrennt sind.

8. Verfahren gemäß einem der Ansprüche 1 bis 7, **dadurch gekennzeichnet, dass** die elektrochemische Oxidation der wässrigen Chlorwasserstoff-LÖsung gemäß Schritt (g) in einer Elektrolysezelle erfolgt, in welcher der Anodenraum und der Kathodenraum durch ein Diaphragma getrennt sind.

9. Verfahren gemäß einem der Ansprüche 1 bis 8, **dadurch gekennzeichnet, dass** die Anode und/oder die Kathode Graphit enthalten.

10. Verfahren gemäß Anspruch 9, **dadurch gekennzeichnet, dass** die Kathode aus Graphit besteht und eine Beschichtung aufweist, welche Iridium enthält.

11. Verfahren gemäß einem der Ansprüche 9 oder 10, **dadurch gekennzeichnet, dass** die Anode und/oder Kathode vertikal angeordnete Nuten aufweist.

12. Verfahren gemäß einem der Ansprüche 1 bis 11, **dadurch gekennzeichnet, dass** der wässrigen Chlorwasserstoff-Lösung vor der elektrochemischen Oxidation gemäß Schritt (e) Metallionen aus der Gruppe der Platinmetalle, vorzugsweise Platin und/oder Palladium, zugesetzt werden.

13. Verfahren gemäß einem der Ansprüche 1 bis 7, **dadurch gekennzeichnet, dass** die Kathode eine Gasdiffusionselektrode ist.

14. Verfahren gemäß einem der Ansprüche 1 bis 7 und 13, **dadurch gekennzeichnet, dass** die Gasdiffusionselektrode wenigstens aus einem elektrisch leitfähigen Gewebe, Geflecht, Gewirke, Netz oder Vlies aus Kohlenstoff besteht, welches zwischen einer kohlenstoffhaltigen Katalysatorschicht und einer Gasdiffusionsschicht angeordnet ist.

15. Verfahren gemäß Anspruch 14, **dadurch gekennzeichnet, dass** die Katalysatorschicht Rhodium und/oder ein Rhodiumsulfid oder eine Mischung aus Rhodium und einem Rhodiumsulfid enthält.

16. Verfahren gemäß einem der Ansprüche 1 bis 7 und 13 bis 15, **dadurch gekennzeichnet, dass** die Anode aus Titan besteht und eine Beschichtung aus wenigstens einem Edelmetalloxid, vorzugsweise Rutheniumoxid, aufweist.

17. Verfahren gemäß einem der Ansprüche 1 bis 7 und 13 bis 16, **dadurch gekennzeichnet, dass** die Elektrolysezelle aus Titan und/oder einer Titanlegierung besteht.

18. Verfahren gemäß einem der Ansprüche 1 bis 7 und 13 bis 17, **dadurch gekennzeichnet, dass** das bei der Herstellung von Phosgen gemäß Schritt (a) eingesetzte Kohlenmonoxid durch Umsetzung von Methan mit Wasser in einem Steamreformer hergestellt wird und der dabei anfallende Wasserstoff mit wenigstens einer organischen Nitroverbindung zu wenigstens einem Amin umgesetzt, welches bei der Herstellung des Isocyanats gemäß Schritt (b) eingesetzt wird.

19. Verfahren gemäß einem der Ansprüche 1 bis 18, **dadurch gekennzeichnet, das** brom- und/oder jodhaltiges Chlor gemäß Schritt (a) zu Phosgen umgesetzt und das so erhaltene Phosgen zur Umsetzung mit TDA gemäß Schritt (b) eingesetzt wird.

## Claims

1. Process for producing isocyanates, comprising the following steps:
(a) Production of phosgene by reacting chlorine with carbon monoxide
(b) Reaction of the phosgene formed according to step (a) with at least one organic amine to form at least one isocyanate and hydrogen chloride
(c) Separation and recovery of the isocyanates formed in step (b)
(d) Separation of the hydrogen chloride formed according to step (b)
(e) Preparation of an aqueous solution of the hydrogen chloride
(f) Purification of the aqueous solution of hydrogen chloride by stripping with steam, such that the proportion of organic solvents is a maximum of 0.05 wt.%
(g) Electrochemical oxidation of at least a part of the aqueous hydrogen chloride solution to form chlorine
(h) Return of at least a part of the chlorine produced according to step (g) to the production of phosgene according to step (a).

2. Process according to claim 1, **characterised in that** the separation according to step (d) of the hydrogen chloride formed according to step (b) involves a separation of phosgene by liquefaction.

3. Process according to claim 1 or 2, **characterised in that** according to step (d) a purification of the hydrogen chloride occurs by means of freezing out.

4. Process according to one of claims 1 to 3, **characterised in that** the preparation of the aqueous hydrogen chloride solution according to step (e) takes place by absorption in an aqueous solution of hydrogen chloride, preferably with a concentration of 15 to 20 wt.%.

5. Process according to one of claims 1 to 4, **characterised in that** according to step (f) a removal of iron, silicon and/or aluminium compounds from the aqueous hydrogen chloride solution additionally takes place by means of chelating ion exchangers.

6. Process according to claim 5, **characterised in that** the aqueous hydrogen chloride solution is an at least 8 wt.% solution.

7. Process according to one of claims 1 to 6, **characterised in that** the electrochemical oxidation of the aqueous hydrogen chloride solution according to step (g) takes place in an electrolytic cell in which the anode chamber and the cathode chamber are separated by an ion-exchange membrane.

8. Process according to one of claims 1 to 7, **characterised in that** the electrochemical oxidation of the aqueous hydrogen chloride solution according to step (g) takes place in an electrolytic cell in which the anode chamber and the cathode chamber are separated by a diaphragm.

9. Process according to one of claims 1 to 8, **characterised in that** the anode and/or the cathode contain graphite.

10. Process according to claim 9, **characterised in that** the cathode consists of graphite and has a coating which contains iridium.

11. Process according to one of claims 9 or 10, **characterised in that** the anode and/or cathode has vertically arranged grooves.

12. Process according to one of claims 1 to 11, **characterised in that** metal ions from the group of platinum metals, preferably platinum and/or palladium, are added to the aqueous hydrogen chloride solution before the electrochemical oxidation according to step (e).

13. Process according to one of claims 1 to 7, **characterised in that** the cathode is a gas diffusion electrode.

14. Process according to one of claims 1 to 7 and 13, **characterised in that** the gas diffusion electrode consists of at least one electrically conductive woven fabric, interwoven fabric, knitted fabric, lattice or nonwoven fabric made from carbon, which is positioned between a carbon-containing catalyst layer and a gas diffusion layer.

15. Process according to claim 14, **characterised in that** the catalyst layer contains rhodium and/or a rhodium sulfide or a mixture of rhodium and a rhodium sulfide.

16. Process according to one of claims 1 to 7 and 13 to 15, **characterised in that** the anode consists of titanium and has a coating of at least one noble metal oxide, preferably ruthenium oxide.

17. Process according to one of claims 1 to 7 and 13 to 16, **characterised in that** the electrolytic cell consists of titanium and/or a titanium alloy.

18. Process according to one of claims 1 to 7 and 13 to 17, **characterised in that** the carbon monoxide used in the production of phosgene according to step (a) is produced by reacting methane with water in a steam reformer and the hydrogen that is produced in this process is reacted with at least one organic nitro compound to form at least one amine, which is used in the production of the isocyanate according to step (b).

19. Process according to one of claims 1 to 18, **characterised in that** chlorine containing bromine and/or iodine is reacted according to step (a) to form phosgene and the phosgene obtained in this way is used for reaction with TDA according to step (b).

## Revendications

1. Procédé de production d'isocyanates comprenant les étapes suivantes :
(a) production de phosgène par réaction du chlore avec le monoxyde de carbone
(b) réaction du phosgène formé selon l'étape (a) avec au moins un amine organique avec formation d'au moins un isocyanate et de chlorure d'hydrogène
(c) séparation et traitement des isocyanates formés dans l'étape (b)
(d) séparation du chlorure d'hydrogène formé selon l'étape (b)
(e) production d'une solution aqueuse du chlorure d'hydrogène
(f) purification de la solution aqueuse de chlorure d'hydrogène par strippage au moyen de vapeur d'eau de sorte que la proportion de solvants organiques est d'au maximum 0,05 % en poids,
(g) oxydation électrochimique d'au moins une partie de la solution aqueuse de chlorure d'hydrogène en chlore
(h) recyclage d'au moins une partie du chlore produit selon l'étape (g) dans la production de phosgène selon l'étape (a).

2. Procédé selon la revendication 1 **caractérisé en ce que** la séparation selon l'étape (d) du chlorure d'hydrogène formé selon l'étape (b) comprend une séparation du phosgène par liquéfaction.

3. Procédé selon la revendication 1 ou 2 **caractérisé en ce que**, selon l'étape (d), une purification du chlorure d'hydrogène par congélation a lieu.

4. Procédé selon l'une des revendications 1 à 3 **caractérisé en ce que** la production de la solution aqueuse de chlorure d'hydrogène selon l'étape (e) a lieu par absorption dans une solution aqueuse de chlorure d'hydrogène, de préférence d'une concentration de 15 à 20 % en poids.

5. Procédé selon l'une des revendication 1 à 4 **caractérisé en ce que**, selon l'étape (f), en outre une élimination des composés du fer, du silicium et/ou de l'aluminium de la solution aqueuse de chlorure d'hydrogène a lieu au moyen d'échangeurs d'ions chélatants.

6. Procédé selon la revendication 5 **caractérisé en ce que** la solution aqueuse de chlorure d'hydrogène est une solution à au moins 8 % en poids.

7. Procédé selon l'une des revendications 1 à 6 **caractérisé en ce que** l'oxydation électrochimique de la solution aqueuse de chlorure d'hydrogène selon l'étape (g) a lieu dans une cellule d'électrolyse dans laquelle le compartiment anodique et le compartiment cathodique sont séparés par une membrane échangeuse d'ions.

8. Procédé selon l'une des revendications 1 à 7 **caractérisé en ce que** l'oxydation électrochimique de la solution aqueuse de chlorure d'hydrogène selon l'étape (g) a lieu dans une cellule d'électrolyse dans laquelle le compartiment anodique et le compartiment cathodique sont séparés par un diaphragme.

9. Procédé selon l'une des revendications 1 à 8 **caractérisé en ce que** l'anode et/ou la cathode contiennent du graphite.

10. Procédé selon la revendication 9 **caractérisé en ce que** la cathode consiste en graphite et comporte un revêtement qui contient de l'iridium.

11. Procédé selon l'une des revendications 9 et 10 **caractérisé en ce que** l'anode et/ou la cathode comportent des rainures disposées verticalement.

12. Procédé selon l'une des revendications 1 à 11 **caractérisé en ce que** des ions métalliques du groupe des métaux du platine, de préférence le platine et/ou le palladium, sont ajoutés à la solution aqueuse de chlorure d'hydrogène avant l'oxydation électrochimique selon l'étape (e).

13. Procédé selon l'une des revendications 1 à 7 **caractérisé en ce que** la cathode est une électrode à diffusion gazeuse.

14. Procédé selon l'une des revendications 1 à 7 et 13 **caractérisé en ce que** l'électrode à diffusion gazeuse consiste au moins en un tissu, treillis, maillage, réseau ou non tissé en carbone électriquement conducteur qui est disposé entre une couche de catalyseur contenant du carbone et une couche à diffusion gazeuse.

15. Procédé selon la revendication 14 **caractérisé en ce que** la couche de catalyseur contient du rhodium et/ou un sulfure de rhodium ou un mélange de rhodium et d'un sulfure de rhodium.

16. Procédé selon l'une des revendications 1 à 7 et 13 à 15 **caractérisé en ce que** l'anode consiste en titane et comporte un revêtement en au moins un oxyde de métal noble, de préférence en oxyde de ruthénium.

17. Procédé selon l'une des revendications 1 à 7 et 13 à 16 **caractérisé en ce que** la cellule d'électrolyse consiste en titane et/ou en un alliage du titane.

18. Procédé selon l'une des revendications 1 à 7 et 13 à 17 **caractérisé en ce que** le monoxyde de carbone utilisé lors de la production du phosgène selon l'étape (a) est produit par réaction du méthane avec l'eau dans un reformeur à la vapeur et l'hydrogène ainsi formé est converti avec au moins un composé nitré organique en au moins une amine qui est utilisée lors de la production de l'isocyanate selon l'étape (b).

19. Procédé selon l'une des revendications 1 à 18 **caractérisé en ce que** du chlore contenant du brome et/ou de l'iode est converti en phosgène selon l'étape (a) et le phosgène ainsi obtenu est utilisé pour la conversion selon l'étape (b) avec de la TDA.
